(19) Europäisches Patentamt
European Patent Office
Office européen des brevets

(11) **EP 2 329 879 A1**

(12) **EUROPEAN PATENT APPLICATION**
published in accordance with Art. 153(4) EPC

(43) Date of publication:
08.06.2011 Bulletin 2011/23

(51) Int Cl.:
*B01J 23/644* (2006.01)  *C07C 5/09* (2006.01)
*C07C 7/167* (2006.01)  *C07C 11/167* (2006.01)
*C07B 61/00* (2006.01)

(21) Application number: 08877066.4

(22) Date of filing: 25.09.2008

(86) International application number:
PCT/JP2008/067355

(87) International publication number:
WO 2010/035325 (01.04.2010 Gazette 2010/13)

(84) Designated Contracting States:
AT BE BG CH CY CZ DE DK EE ES FI FR GB GR HR HU IE IS IT LI LT LU LV MC MT NL NO PL PT RO SE SI SK TR
Designated Extension States:
AL BA MK RS

(71) Applicant: Sued-Chemie Catalysts Japan, Inc.
Shibuya-ku
Tokyo 151-0053 (JP)

(72) Inventors:
• SUGETA, Moriyasu
Toyama-shi
Toyama 939-2753 (JP)
• FUKADA, Hirofumi
Tokyo 151-0053 (JP)

(74) Representative: Splanemann, Rainer
Splanemann
Patentanwälte Rechtsanwalt Partnerschaft
Rumfordstrasse 7
80469 München (DE)

(54) **CATALYST FOR SELECTIVE HYDROGENATION OF ACETYLENE COMPOUNDS IN 1,3-BUTADIENE, METHOD FOR PRODUCING THE SAME AND METHOD OF USING THE SAME**

(57) The present invention provides a catalyst for selective hydrogenation used in a process for obtaining 1,3-butadiene by selectively hydrogenating acetylene compounds contained in a C4 hydrocarbon compound reservoir which is obtained by steam cracking and mainly contains 1,3-butadiene. This catalyst composition mainly contains palladium and bismuth, or palladium, bismuth and tellurium.

[Figure 1(a)]

**Description**

TECHNICAL FIELD

The present invention relates to a catalyst employed in a reaction for obtaining highly pure 1,3-butadinene by selectively hydrogenating acetylene compounds contained in a C4 hydrocarbon compound reservoir which mainly contains 1,3-butadiene; a method of producing the catalyst; and a method of using the catalyst.

BACKGROUND ART

A variety of olefin compounds used as basic raw material in the petrochemical industry are usually produced by steam cracking of naphtha. Since such olefin compounds contain highly-unsaturated hydrocarbon compounds, in order to utilize them as a raw material in the macromolecular chemical industry, such hydrocarbon compounds must be removed. In order to produce olefin compounds such as ethylene, propylene, butadiene and isoprene and other similar compounds, it is required to minimize the loss of useful materials in the feed flow, such as ethylene, propylene, butanes, butadiene, isoprene and other similar compounds, and to remove various acetylene-type impurities such as acetylene, methylacetylene, vinylacetylene, ethylacetylene; 2-methyl-1-butane-3-ene and other similar compounds from a mixed reservoir of unpurified C2 to C5 compounds.

Examples of a method of removing such highly-unsaturated hydrocarbon compounds include a method in which these compounds are removed by allowing them to selectively react with hydrogen in the presence of a catalyst and a method of removing these compounds by solvent extraction.

In such methods, it is known that many metals have an activity for the hydrogenation reaction of highly-unsaturated hydrocarbon compound. In the case of hydrogenation reaction carried out in the petrochemical industry to purify olefin compounds, a highly selective catalyst is required in order to prevent the loss of olefins caused by hydrogenation of olefin compounds which is a side reaction, and as an appropriate catalyst therefor, a palladium-based catalyst is used.

Such palladium-based catalyst used in the selective hydrogenation reaction has not only high selectivity, but also excellent activity; therefore, by adjusting the reaction temperature, the catalyst is used while inhibiting side reactions and unreacted highly-unsaturated hydrocarbon compounds. Yet, when comparing the reactivity for olefin compounds and acetylene compounds, the palladium-based catalyst has markedly higher reactivity for acetylene compounds.

However, since the amount of olefin compounds normally existing in the reaction system is overwhelmingly greater than that of acetylene compounds, hydrogenation of olefin compounds also occurs along with hydrogenation of acetylene compounds. From the standpoint of the yield of a desired compound, it is preferred that the catalyst allow minimal amount of this hydrogenation reaction of olefin compounds as side reaction. Further, as another side reaction, precipitation of carbonaceous substances also occurs on the catalyst due to polymerization of highly-unsaturated compounds. It is, therefore, also preferred that such precipitation of polymerized substances be minimized as much as possible since it can reduce the regeneration rate of the catalyst.

Especially, it is difficult to remove trace amounts of C4 acetylene compounds such as vinylacetylene and ethylacetylene from a C4 hydrocarbon reservoir mainly containing 1,3-butadiene by hydrogenation, and butadiene is also hydrogenated to generate butane. However, since butadiene is useful as a polymer raw material and the loss thereof leads to a large economical loss, it is preferred to minimize such loss as much as possible.

So far, as a method of improving the performance of a palladium-based catalyst, it is reported in Patent Document 1 that the selectivity is improved by adding Ag to the catalyst composition. In addition, Patent Document 2 reports that the selectivity is improve by adding Ag and Bi to Pd in the catalyst composition. Further, Patent Document 3 reports a catalyst in which Te or Sb was added in order to suppress the elution of Pd into highly-concentrated butadiene flow. However, while the suppression of Pd elution is described therein, there is no description regarding improvement of the catalyst selectivity. That is, none of the prior arts could completely remove C4 acetylenes while maintaining the activity of the palladium-based catalyst for selective hydrogenation of C4 acetylene compounds at a high level and also in terms of the selectivity level, without a loss of 1,3-butadiene caused by excessive hydrogenation.

Patent Document 1: U.S. Patent No. 4547600
Patent Document 2: U.S. Patent No. 6459008
Patent Document 3: Japanese Patent Publication No. S62-23726

DISCLOSURE OF THE INVENTION

PROBLEMS TO BE SOLVED BY THE INVENTION

Those previously reported palladium/alumina catalysts have an improved selectivity by an addition of a co-catalyst component; however, they have practical problems which could not be satisfied by the prior arts. As a catalyst which selectively hydrogenates acetylene compounds contained in a C4 hydrocarbon compound reservoir which was obtained by steam cracking or the like and mainly contains 1,3-butadiene, the present invention was made to solve such problems of the prior arts. Therefore, an object of the present invention is to provide a catalyst having a high selectivity which is sufficiently satisfactory from the practical standpoint, the catalyst selectively hydrogenating trace amounts of acetylene compounds contained in a C4 hydrocarbon compound reservoir mainly containing 1,3-butadiene; a method of producing the catalyst; and a method using the catalyst.

MEANS FOR SOLVING THE PROBLEMS

In order to solve the aforementioned problems, the present inventors intensively studied to discover that a catalyst which hardly hydrogenates 1,3-butadiene, that is, a highly selective catalyst can be produced by adding a novel co-catalyst component to a palladium catalyst and reducing it with hydrogen at a high temperature.
That is, the present invention provides a catalyst hydrogenating acetylene compounds in 1,3-butadiene, which catalyst is characterized by containing palladium and bismuth, or palladium, bismuth and tellurium and performing the hydrogenation at a high temperature; a method of producing the catalyst; and a method of using the catalyst.

EFFECTS OF THE INVENTION

By using the catalyst according to the present invention, selective hydrogenation reaction of acetylene compounds such as vinylacetylene and ethylacetylene which are contained at a trace amount in a C4 hydrocarbon compound reservoir mainly containing 1,3-butadiene can be carried out without losing 1,3-butadiene due to hydrogenation; therefore, a catalyst having much higher selectivity compared to conventional catalysts; a method of producing the catalyst; and a method of using the catalyst can be provided.

BRIEF DESCRIPTION OF THE DRAWINGS

[Fig. 1(a)]

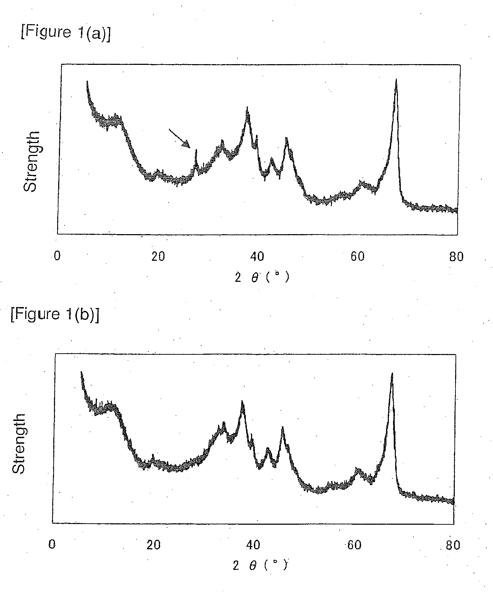

Fig. 1(a) shows the physical property of the catalyst according to Example 1 measured by an XRD measuring apparatus.
[Fig. 1(b)]
Fig. 1 (b) shows the physical property of the catalyst according to Comparative Example 4 measured by an XRD measuring apparatus.

BEST MODE FOR CARRYING OUT THE INVENTION

The present invention will now be described in more detail.
The catalyst according to the present invention can be prepared by allowing an inorganic carrier to support a palladium compound; treating the inorganic carrier with a reducing agent, followed by drying or calcinating in the air; introducing thereto a metal compound; drying or calcinating the resultant; and subsequently reducing it at a high temperature in an air flow containing hydrogen.
The palladium raw material of the catalyst composition according to the present invention is not particularly restricted as long as it does not contain a component which is degraded by a heat treatment and eventually remains on the catalyst to be a catalyst poison. As such palladium raw material, inorganic and organic compounds, such as palladium chloride, palladium nitrate, tetrachloropalladium and acetonato palladium, may be employed, and the amount of palladium supported on the carrier is in the range of 0.01 to 1% by weight based on the total weight of the catalyst composition.
As the carrier, metal oxides such as alumina, silica, titania and silica-alumina can be used, and alumina is particularly preferred. Alumina may be used in the form of a molded product such as a tableted product, cylindrical product, extruded product or spherical product, and alumina having a surface area in the range of 1 to 3 50 $m^2/g$ may be used.
Palladium is supported onto the carrier by, for example, a soaking method in which a support solution containing palladium is prepared and the carrier is soaked thereinto, a spray method in which a support solution is sprayed to the carrier, or an impregnation method in which a support solution is prepared at an amount comparable to the amount of water absorption by the carrier and the carrier is impregnated with the entire amount of the support solution. After being made to support palladium, the carrier is reduced after drying or calcination thereof or without drying. The reduction is carried out with hydrogen or by liquid-phase reduction. In the liquid-phase reduction, an aqueous solution of a reducing agent such as formalin, sodium formate or hydrogenated boron can be used, and after the reduction, the carrier is washed with water and dried or calcined. Here, the temperature of the calcination or reduction is not particularly restricted; however, it is in the range of room temperature to 600°C.

It is desired that palladium be supported on the carrier in an eggshell structure. When the thickness of the palladium layer from the outer surface of the catalyst is not greater than 400 $\mu$m, the loss of butadiene is small and a high selectivity can be attained.

The metal compound used as co-catalyst component of the present invention contains bismuth and may be used in the form of a nitrate salt thereof, an inorganic salt thereof such as a chloride salt or an organic salt thereof such as an acetic acid salt. The content of the bismuth compound is in the range of approximately 0.01 to 10% by weight, more preferably in the range of 0.1 to 6% by weight, based on the total weight of the catalyst composition. The weight ratio of the bismuth compound to the palladium compound is preferably 0.1 to 10, more preferably 0.5 to 8.

As the co-catalyst component according to the present invention, a tellurium compound may be further added to the bismuth compound. The tellurium compound may be used in the form of an oxoacid such as a telluric acid salt, an inorganic salt such as a chloride salt, or an oxide. The content of the tellurium compound is in the range of approximately 0.01 to 0.1% by weight, more preferably at an amount of approximately 0.01 to 0.05% by weight, based on the total weight of the catalyst composition.

The co-catalyst component is supported onto the carrier in the same manner as in the case of palladium, for example, by a soaking method, spray method or impregnation method. The aforementioned metal compounds may be supported onto the carrier simultaneously with palladium, or the co-catalyst component may also be further added to the carrier already supporting palladium.

After being made to support these metal catalyst components, the carrier is dried or calcinated, and then reduced in hydrogen gas flow. Here, the calcination temperature is not particularly restricted; however, it is in the range of room temperature to 800°C.

The gas used in the reduction which is an important feature of the present invention may also contain an inert gas such as nitrogen in addition to hydrogen. The reduction may be carried out at a temperature in the range of 250 to 600°C, and it is preferably carried out at a temperature in the range of 300 to 500°C. This reduction carried out in hydrogen gas flow at a high temperature is critical, and at approximately 150°C, which is a temperature normally used for reduction, the selectivity is reduced. In addition, even with an addition of silver to the catalyst component as described in Patent Document 2, a practically sufficient selectivity cannot be attained by hydrogen reduction at a low temperature. The reason why a high selectivity is attained by the reduction at a high temperature is believed to be the generation of compounds such as PdBi and PdBi$_2$ due to alloying of Pd and the co-catalyst component caused by the reduction at a high temperature.

As a result of the analyses on the catalyst physical properties by X-ray diffraction (XRD) analysis, when the catalyst according to the present invention containing Pd and the co-catalyst component was reduced at a high temperature, there was a novel peak which was not observed when the catalyst was reduced at a low temperature; therefore, it is believed that an alloy of Pd and the co-catalyst component, Bi, was generated by the reduction at a high temperature. Since this XRD measurement is based on the crystalline regularity, information regarding the crystal such as strain and size can be obtained in addition to the crystal quality. The presence of a different peak indicates a change in the crystal structure even in the same composition. For example, generation of an alloy such as PdBi$_2$ results in a peak at such a position indicating the aforementioned change. In this manner, the catalyst according to the present invention is intrinsically different from those known catalysts in that an active component of alloyed metal is generated by carrying out reduction of the catalyst at a high temperature in hydrogen gas flow. This novel catalyst having such physical property selectively hydrogenates trace amounts of C4 acetylenes such as vinylacetylene and ethylacetylene that are contained in a C4 hydrocarbon compound reservoir containing a large amount of 1,3-butadiene.

For the selective hydrogenation reaction using the catalyst according to the present invention, either of a fixed-bed-type reactor or fluidized-bed-type reactor may be used, and the reaction may also be carried out in any of a gas phase, liquid phase or mixed phase. In the gas phase reaction, for example, the ratio of the olefin compounds existing at the reactor outlet can be increased by feeding via the reactor inlet using a carrier gas a mixture of an acetylene compound and an olefin compound such as 1,3-butadiene, as well as hydrogen gas, into the reactor to which the catalyst according to the present invention has already been loaded.

The C4 hydrocarbon compound reservoir mainly containing 1,3-butadiene in which the catalyst according to the present invention is used refers to a compound reservoir which contains butane, isobutane, butene, isobutene, 1,3-butadiene, 1,2-butadiene, ethylacetylene, vinylacetylene and small amounts of C3 and C5 hydrocarbons, and it mainly contains 1,3-butadiene at an amount of 30 to 60% and vinylacetylene and ethylacetylene at an amount of 0.1 to 5%.

Further, the selective hydrogenation reaction in which the catalyst according to the present invention is used is optimally carried out under the conditions of: the reaction temperature at 40 to 100°C; the pressure at 0.5 to 5 MPa; LHSV = 1 to 20h-1; and hydrogen/acetylenes = 0.5 to 2.

EXAMPLES

The method according to the present invention will now be described by way of representative examples thereof; however, the present invention is not restricted thereto.

Example 1

Added to 1,200 ml of pure water was 18.3 ml of palladium chloride solution (120 g/L), and 1,000 g of alumina carrier was further added thereto. The thus obtained mixture was left to stand for 90 minutes and after removing the solution, the resultant was reduced with 5% Na formate solution at 60°C, washed with water and then dried in the air to obtain a Pd catalyst. On another front, 4.3 g of bismuth nitrate was completely dissolved in a solution obtained by adding 15 ml of concentrated nitric acid to 165 ml of pure water. The thus obtained solution was added to the Pd catalyst (300 g), and the resultant was calcinated at 500°C and subsequently reduced in hydrogen at 350°C to obtain a catalyst. This catalyst had a composition ratio of Pd/Bi=0.2%/0.6%.

This catalyst was subjected to a measurement by an XRD measuring apparatus. The measurement was carried out by using a copper anticathode at an output of 40 mA and 45 kV and a wavelength of 1.54 angstrom. The result is shown in Fig. 1(a). There was a peak at about 2 =28°, which was not observed in Fig. 1(b) showing peaks for the later-described catalyst according to Comparative Example 4.

Example 2

A Pd catalyst was obtained in the same manner as in Example 1. On another front, 2.9 g of bismuth nitrate was completely dissolved in a solution obtained by adding 10 ml of concentrated nitric acid to 170 ml of pure water. The thus obtained solution was added to the Pd catalyst (300 g), and the resultant was calcinated at 500°C and subsequently reduced in hydrogen at 350°C to obtain a catalyst. This catalyst had a composition ratio of Pd/Bi = 0.2%/0.4%.

Example 3

A Pd catalyst was obtained in the same manner as in Example 1. On another front, 5.6 g of bismuth nitrate was completely dissolved in a solution obtained by adding 20 ml of concentrated nitric acid to 160 ml of pure water. The thus obtained solution was added to the Pd catalyst (300 g), and the resultant was calcinated at 500°C and subsequently reduced in hydrogen at 350°C to obtain a catalyst. This catalyst had a composition ratio of Pd/Bi = 0.2%/0.8%.

Example 4

To 200 g of sodium chloride aqueous solution (1.2% by weight), 3.7 g of palladium chloride powder was dissolved and pure water was added to obtain 1,200 g of palladium aqueous solution. After making the temperature of this solution to be 60°C, 1,000 g of alumina carrier was added and the resulting solution was left to stand for 90 minutes. After removing the solution, the resultant was reduced with 5% Na formate solution at 60°C, washed with water and then dried in the air to obtain a Pd catalyst. On another front, 10 g of bismuth nitrate was completely dissolved in a solution obtained by adding 35 ml of concentrated nitric acid to 145 ml of pure water. The thus obtained solution was added to the Pd catalyst (300 g), and the resultant was calcinated at 500°C and reduced in hydrogen at 350°C to obtain a catalyst. This catalyst had a composition ratio of Pd/Bi = 0.2%/1.4%.

Example 5

Added to 1,200 ml of pure water was 18.3 ml of palladium chloride solution (120 g/L), and 1,000 g of alumina carrier was further added thereto. The thus obtained mixture was left to stand for 90 minutes and after removing the solution, the resultant was put into 1,000 ml of 5% Na formate solution at 60°C, left to stand for 3 hours, washed with water and then dried in the air to obtain a Pd catalyst. On another front, 4.3 g of bismuth nitrate and 0.162 g of telluric acid were completely dissolved in a solution obtained by adding 15 ml of concentrated nitric acid to 165 ml of pure water. The thus obtained solution was added to the Pd catalyst (300 g), and the resultant was calcinated at 500°C and reduced in hydrogen at 350°C to obtain a catalyst. This catalyst had a composition ratio of Pd/Bi/Te = 0.2%/0.6%/0.02%.

Comparative Example 1

Added to 1,200 ml of pure water was 18.3 ml of palladium chloride solution (120 g/L), and 1,000 g of alumina carrier was further added thereto. The thus obtained mixture was left to stand for 90 minutes and after removing the solution, the resultant was put into 1,000 ml of 5% Na formate solution at 60°C, left to stand for 3 hours, washed with water and then dried in the air to obtain a Pd catalyst. On another front, 0.47 g of silver nitrate was completely dissolved in 180 ml of pure water, and after adding the thus obtained solution to the Pd catalyst (300 g), the resultant was calcinated at 500°C to obtain a catalyst. This catalyst had a composition ratio of Pd/Ag = 0.2%/0.1%.

Comparative Example 2

Added to 1,200 ml of pure water was 18.3 ml of palladium chloride solution (120 g/L), and 1,000 g of alumina carrier was further added thereto. The thus obtained mixture was left to stand for 90 minutes and after removing the solution, the resultant was put into 1,000 ml of 5% Na formate solution at 60°C, left to stand for 3 hours, washed with water and then dried in the air to obtain a Pd catalyst. On another front, 4.3 g of bismuth nitrate was completely dissolved in a solution obtained by adding 15 ml of concentrated nitric acid to 165 ml of pure water. The thus obtained solution was added to the Pd catalyst (300 g) and the resultant was calcinated at 500°C to obtain a PD/Bi catalyst. Further, after completely dissolving 0.14 g of silver nitrate in 180 ml of pure water, the thus obtained solution was added to the Pd/Bi catalyst (300 g), and the resultant was calcinated at 500°C to obtain a catalyst. This catalyst had a composition ratio of Pd/Bi/Ag = 0.2%/0.6%/0.03%.

Comparative Example 3

Added to 1,200 ml of pure water was 18.3 ml of palladium chloride solution (120 g/L), and 1,000 g of alumina carrier was further added thereto. The thus obtained mixture was left to stand for 90 minutes and after removing the solution, the resultant was put into 1,000 ml of 5% Na formate solution at 60°C, left to stand for 3 hours, washed with water and then dried in the air to obtain a Pd catalyst. On another front, after completely dissolving 0.16 g of telluric acid in 180 ml of pure water, the thus obtained solution was added to the Pd catalyst (300 g), and the resultant was calcinated at 500°C to obtain a catalyst. This catalyst had a composition ratio of Pd/Te = 0.2%/0.03%.

Comparative Example 4

A catalyst was prepared in the same manner as in Example 1 except for the reduction temperature. The reduction was carried out with hydrogen at 150°C. This catalyst had a composition ratio of Pd/Bi = 0.2%/0.6%.

This catalyst was subjected to a measurement by an XRD measuring apparatus. The measurement was carried out by using a copper anticathode at an output of 40 mA and 45 kV and a wavelength of 1.54 angstrom. The result is shown in Fig. 1 (b). As clearly seen from the figure, there was no peak observed at about 2 = 28°, which was observed for the catalyst according to Example 1.

Test Example

Using the catalysts according to Examples 1 and 2 and Comparative Examples 1 to 4, their performances of selectively hydrogenating acetylene compounds were evaluated under the following reaction conditions.

(Test Conditions)

Amount of catalyst = 30ml
LHSV = 10h-1
Pressure = 2.9 MPa
Reaction temperature = 35°C
Hydrogen / acetylene = 1.0
Recycle ratio = 0.5 to 1.0
The reaction ratio is a ratio at which the post-reaction solution is mixed with the raw material to dilute the raw material.
Recycle ratio = Amount of post-reaction solution (ml/h) / amount of raw material solution (ml/h)

(Composition of raw material)

Vinylacetylene = 0.3 to 0.5%
Ethylacetylene = 0.13 to 1.16%
1,3-butadiene = 44%
1-butene = 18%
t-2-butene = 5 %
c-2-butene = 4%
i-butene = 24%
N-butane = 4%

(Measurement Method)

The degree of acetylene conversion was determined by measuring the concentrations of vinylacetylene and ethylacetylene in the solution before and after the reaction by gas chromatography and using the following equation.
[Formula 1]

$$\text{Degree of acetylene conversion} = \{(\triangle\text{vinylacetylene} + \triangle\text{ethylacetylene}) / (\text{inlet vinylacetylene} + \text{inlet ethylacetylene})\} \times 100(\%)$$

$$\triangle\text{vinylacetylene} = \text{inlet vinylacetylene} - \text{outlet vinylacetylene}$$

$$\triangle\text{ethylacetylene} = \text{inlet ethylacetylene} - \text{outlet ethylacetylene}$$

The ratio of butadiene loss was determined by measuring the concentration of 1,3-butadiene in the raw material and the post-reaction solution and using the following equation.
[Formula 2]

$$\text{Butadiene loss} = (\triangle\,1,3\text{-butadiene} / \text{inlet } 1,3\text{-butadiene}) \times 100(\%)$$

$$\triangle\,1,3\text{-butadiene} = \text{inlet } 1,3\text{-butadiene} - \text{outlet } 1,3\text{-butadiene}$$

The test results are shown in Table 1. When compared at the same degree of C4 acetylene conversion, the catalysts according to Examples 1 and 2 resulted in a smaller 1,3-butadiene loss than the catalysts according to Comparative Examples 1 to 4, and the catalysts according to Examples had a higher selectivity.
[Table 1]

| | Recycle=0 | | Recycle=0.5 | | Recycle=1.0 | |
|---|---|---|---|---|---|---|
| | Degree of C4 acetylene conversion (%) | Ratio of butadiene loss (%) | Degree of C4 acetylene conversion (%) | Ratio of butadiene loss (%) | Degree of C4 acetylene conversion (%) | Ratio of butadiene loss (%) |
| Example 1 | 60.4 | -0.7 | 72.0 | 0.2 | 83.8 | 2.2 |
| Example 2 | 55.4 | -1.2 | 67.9 | -0.9 | 77.5 | 0.9 |
| Example 3 | 56.2 | -1.1 | 75.1 | -0.6 | 82.7 | 0.5 |
| Example 4 | 64.0 | -0.6 | 77.2 | 0.5 | 82.5 | 2.2 |
| Example 5 | 51.8 | -1.0 | 70.3 | 0.5 | 79.6 | 3.4 |
| Comparative Example 1 | 32.0 | -0.3 | 50.5 | 2.3 | 59.3 | 4.1 |
| Comparative Example 2 | 53.2 | -0.1 | 64.0 | 0.9 | 72.1 | 2.7 |
| Comparative Example 3 | 48.1 | 1.3 | 60.3 | 2.4 | 74.7 | 4.0 |
| Comparative Example 4 | 41.8 | -1.1 | 57.1 | 0.4 | 66.0 | 1.2 |

INDUSTRIAL APPLICABILITY

The catalyst for selective hydrogenation according to the present invention can selectively hydrogenate trace amounts of acetylene hydrocarbons contained in a hydrocarbon reservoir obtained by steam cracking or the like, particularly in a C4 hydrocarbon compound reservoir from which separation of acetylene hydrocarbons was conventionally very difficult since a large amount of 1,3-butadiene is contained. At the same time, the catalyst for selective hydrogenation according to the present invention can prevent excessive hydrogenation reaction. Therefore, it minimizes the loss of useful resources and is very advantageous in industrial cracking processes. Further, the catalyst according to the present invention can drastically reduce the cost for producing a final raw material for macromolecular chemical industry.

**Claims**

1.  A catalyst for selective hydrogenation of acetylene compounds in a C4 hydrocarbon compound reservoir containing 1,3-butadiene, said catalyst being **characterized by** containing a palladium compound supported by an inorganic carrier and a metal compound other than palladium and being reduced in an air flow containing hydrogen.

2.  The catalyst according to claim 1, **characterized in that** said metal compound is composed of a bismuth compound or a bismuth compound and a tellurium compound.

3.  The catalyst according to claim 2, **characterized in that** the content of said palladium compound is 0.01 to 1% by weight and the content of said bismuth compound is 0.01 to 10% based on the total catalyst weight, and that the weight ratio of said bismuth compound to said palladium compound is 0.1 to 10.

4.  The catalyst according to claim 3, **characterized in that** the content of said tellurium compound is 0.01 to 0.1% by weight based on the total catalyst weight.

5.  The catalyst according to claim 1, **characterized by** being reduced at a temperature of 250 to 600°C.

6.  The catalyst according to claim 1, **characterized in that** said C4 hydrocarbon compound reservoir contains said 1,3-butadiene at an amount of 30 to 60% and said acetylene compound at an amount of 0.1 to 5%.

7. A method of producing the catalyst according to any one of claims 1 to 6, **characterized in that** said catalyst is produced by allowing an inorganic carrier to support a palladium compound; treating said inorganic carrier with a reducing agent; drying or calcinating the resulting carrier in the air; introducing thereto a metal compound; drying or calcinating the resultant; and subsequently reducing it in an air flow containing hydrogen at a high temperature.

8. A method of using the catalyst according to claim 1, said catalyst being used for selectively hydrogenating acetylene compounds in a C4 hydrocarbon compound reservoir containing 1,3-butadiene to convert said acetylene compound to an olefin compound.

[Figure 1(a)]

[Figure 1(b)]

<div align="center">

**INTERNATIONAL SEARCH REPORT**

</div>

| | International application No. |
|---|---|
| | PCT/JP2008/067355 |

A. CLASSIFICATION OF SUBJECT MATTER
*B01J23/644*(2006.01)i, *C07C5/09*(2006.01)i, *C07C7/167*(2006.01)i, *C07C11/167* (2006.01)i, *C07B61/00*(2006.01)n

According to International Patent Classification (IPC) or to both national classification and IPC

B. FIELDS SEARCHED

Minimum documentation searched (classification system followed by classification symbols)
B01J23/644, C07C5/09, C07C7/167, C07C11/167, C07B61/00

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2005-512785 A  (Sud-Chemie, Inc.),<br>12 May, 2005 (12.05.05),<br>Claims; Par. Nos. [0031] to [0035]; examples<br>& US 2003/0134744 A1    & EP 1458480 A1<br>& WO 2003/053574 A1 | 1-6,8<br>7 |
| Y | JP 2007-531614 A  (Catalytic Distillation Technologies),<br>08 November, 2007 (08.11.07),<br>Par. Nos. [0002] to [0005]<br>& US 2005/0209491 A1    & WO 2005/094418 A2 | 1-6,8 |

☒ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 04 December, 2008 (04.12.08) | 16 December, 2008 (16.12.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

| International application No. |
| --- |
| PCT/JP2008/067355 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | JP 62-023726 B2  (Japan Synthetic Rubber Co., Ltd.), 25 May, 1987 (25.05.87), Page 1, column 1, line 19 to page 1, column 2, line 3; page 3, column 5, the last line to page 3, column 6, line 15; referential example 2 (Family: none) | 2,4,6 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4547600 A
- US 6459008 B
- JP S6223726 B